# FASCICULE DE BREVET EUROPEEN

(11) **EP 2 219 678 B1**
(45) Date de publication et mention de la délivrance du brevet: **19.05.2021**
(21) Numéro de dépôt: 08856747.4
(22) Date de dépôt: 27.11.2008
(51) Int. Cl.: A61K 47/54, A61K 47/69, A61P 3/10, A61P 35/00, A61P 37/06

(54) **NANOPARTICULES D'ACTIFS THERAPEUTIQUES DE FAIBLE SOLUBILITE AQUEUSE**
NANOPARTIKEL VON WIRKSTOFFEN GERINGER WASSERLÖSLICHKEIT
NANOPARTICLES OF THERAPEUTIC AGENTS HAVING LOW WATER SOLUBILITY

(30) Priorité: 27.11.2007 FR 0708296
(43) Date de publication de la demande: 25.08.2010
(73) Titulaire: Centre National de la Recherche Scientifique, 75016 Paris (FR); Université Paris Sud XI, 91405 Orsay (FR)
(72) Inventeur: COUVREUR, Patrick, F-91140 Villebon Sur Yvette (FR); LAKKIREDDY, Harivardhan, Reddy, F-92240 Malakoff (FR); DOSIO, Franco, I-10155 Torino (IT); CATTEL, Luigi, I-10020 Pecetto (IT); STELLA, Barbara, 10134 Torino (IT)
(74) Mandataire: IPAZ
(86) Numéro de dépôt international: PCT/FR2008/052147
(87) Numéro de publication internationale: WO 2009/071850

(56) Documents cités:
- WO-A-00/71079
- WO-A-99/12545
- WO-A-2006/090029
- US-A- 5 399 554
- US-A1- 2005 100 577
- COUVREUR PATRICK ET AL: "Squalenoyl nanomedicines as potential therapeutics." NANO LETTERS NOV 2006, vol. 6, no. 11, novembre 2006 (2006-11), pages 2544-2548, XP002489419 ISSN: 1530-6984
- DUAN-YUN SI: "Biomedical evaluation of nanomedicines" ASIAN JOURNAL OF PHARMACODYNAMICS AND PHARMACOKINETICS, [Online] vol. 7, no. 2, 2007, pages 83-97, XP002489470 Hong Kong ISSN: 1608-2281 Extrait de l'Internet: URL:http://www.hktmc.com/ChineseMedia/Maga zine/Medicine/ajdmpk/AJDMPK-2007-2/AJPP200 7-2_83-97_Nanomed.pdf> [extrait le 2008-07-23]
- KINGSTON D G I ET AL: "Taxoids: Cancer-fighting compounds from nature" CURRENT OPINION IN DRUG DISCOVERY AND DEVELOPMENT 200703 GB, vol. 10, no. 2, mars 2007 (2007-03), pages 130-144, XP009103714 ISSN: 1367-6733
- PASUT ET AL: "Polymer-drug conjugation, recent achievements and general strategies" PROGRESS IN POLYMER SCIENCE, PERGAMON PRESS, OXFORD, GB, vol. 32, no. 8-9, 7 août 2007 (2007-08-07), pages 933-961, XP022188828 ISSN: 0079-6700
- National Center for Biotechnology Information: "Deoxyspergualin", PubChem Compound Database , Retrieved from the Internet: URL:https://pubchem.ncbi.nlm.nih.gov/compo und/5458826#section=Top [retrieved on 2018-09-20]
- National Center for Biotechnology Information: "Doxorubicin", PubChem Compound Database , Retrieved from the Internet: URL:https://pubchem.ncbi.nlm.nih.gov/compo und/31703#section=Top [retrieved on 2018-09-20]

## Description

La présente invention vise à proposer des formulations nanoparticulaires hydrodispersibles d'actifs thérapeutiques de faible solubilité aqueuse, voire hydrophobes.

D'une manière générale, un actif est dit faiblement hydrosoluble lorsqu'il présente une solubilité dans l'eau pure inférieure à 100 µg/ml à température ambiante c'est-à-dire une température de 25 °C environ.

Ainsi, comme décrit ci-après, de nombreux actifs thérapeutiques et/ou de candidats thérapeutiques s'avèrent faiblement hydrosolubles, voire totalement hydrophobes ce qui pose des difficultés notables sur le plan de leur formulation thérapeutique. Notamment, il s'avère très difficile de les formuler sous une forme compatible avec une administration par voie systémique.

Certes, leur solubilisation en milieu aqueux peut, pour certains d'entre eux, être acquise sous réserve de les formuler à l'état de sels organiques ou inorganiques.

Toutefois, ces sels ou encore dérivés, ne sont pas, dans la plupart des cas, facilement accessibles en terme de synthèse et, d'autre part, pas toujours souhaités. En effet, leur préparation peut se faire au détriment de la stabilité de la molécule active.

A titre illustratif de ces actifs thérapeutiques peu hydrosolubles, on peut notamment citer les cyclosporines, les taxoïdes, les taxanes et des molécules peptidiques à l'image par exemple de l'insuline.

Les cyclosporines sont des composés anti-fongiques, dotés généralement d'une activité d'immunosuppresseur. Ces cyclosporines manifestent le plus souvent une solubilité aqueuse n'excédant pas 25 µg/ml, soit une valeur qui est approximativement cent fois inférieure à celle requise pour une absorption régulière par l'organisme. Pour obtenir une biodisponibilité acceptable en cyclosporine, les formulations conventionnelles mettent généralement en œuvre des systèmes de dispersion associant une phase hydrophile, une phase hydrophobe et un tensioactif.

Pour leur part, les taxoïdes ou taxanes, connus pour leurs propriétés anti-néoplasiques, sont des substances diterpéniques. Le paclitaxel qui est un taxoïde naturel, et son dérivé semi-synthétique, le docétaxel, sont largement utilisés pour le traitement des tumeurs. Les dérivés de taxane possèdent, d'une manière générale, une solubilité aqueuse encore inférieure à celle des cyclosporines. Ainsi, cette très faible solubilité aqueuse a nécessité l'élaboration de formulations spécifiques. Par exemple, la formulation de paclitaxel, distribuée sous la dénomination TAXOL® par BRISTOL MEYERS SQUIBB et dédiée à une administration par voie systémique, est une formule stérile, non pyrogénique disponible en dose unique contenant 30 mg de docétaxel anhydre. Outre, l'actif, chaque dose contient du triricinolate de polyoxyéthylène glycol, le Crémophore EL® et de l'éthanol. Enfin, ce type de formulation nécessite d'être dilué dans une solution de perfusion (chlorure de sodium 0,9 %, glucose 5 % etc.) stérile, apyrogène et isotonique avant administration.

Or, la présence de Crémophore EL® et d'éthanol, qui sont nécessaires pour pallier au défaut de solubilité du paclitaxel, sont malheureusement de nature à causer des effets indésirables. Ainsi, pour prévenir tout risque de manifestation d'hypersensibilité secondaire, il est généralement procédé à une prémédication du sujet à traiter avec de la dexaméthasone par voie orale, trois jours avant l'initialisation de la chimiothérapie.

Une autre alternative de formulation du paclitaxel met en œuvre le paclitaxel lié à de l'albumine. Cette formulation spécifique est commercialisée sous la dénomination Abraxane®. Pour des raisons évidentes, cette alternative n'est également pas satisfaisante dans la mesure où elle impose la formation de ce dérivé, par liaison covalente d'une molécule d'albumine à une molécule de paclitaxel. Qui plus est, on ne peut totalement exclure que ce couplage ait une incidence sur l'activité thérapeutique du paclitaxel.

Enfin, il est clair que ce défaut de biodisponibilité des actifs faiblement hydrosolubles, devient un handicap exacerbé lorsque l'actif est destiné à être administré par voie orale, autre voie particulièrement appréciée pour l'administration d'actifs thérapeutiques.

DUAN-YUN SI: "Biomédical evaluation of nanomedicines", ASIAN JOURNAL OF PHARMACODYNAMICS AND PHARMACOKINETICS vol. 7, no. 2 2007, pages 83-97, Hong Kong ISSN: 1608-2281 décrit des nanomédicaments tels que le Xyotax à base de paclitaxel polyglutamate, un conjugué hydrosoluble. WO 00/71079 décrit des compositions pharmaceutiques dépourvues de crémophor à base de nanoparticules hydrodispersibles composées de paclitaxel lié à l'albumine. KINGSTON D G I ET AL: "Taxoids: Cancer-fighting compounds from nature", CURRENT OPINION IN DRUG DISCOVERY AND DEVELOPMENT 200703 GB, vol. 10, no. 2, 2007-03, pages 130-144, ISSN: 1367-6733 décrit des conjugués de taxanes avec de l'albumine, du polyglutamate, de l'acide docosahexaénoïque, fullerène. PASUT ET AL: "Polymerdrug conjugation, recent achievements and général stratégies", PROGRESS IN POLYMER SCIENCE, PERGAMON PRESS, OXFORD, GB, vol. 32, no. 8-9, 2007-08-07, pages 933-961, ISSN: 0079-6700 décrit des nanomédicaments solubles dans l'eau à base par ex. de taxanes, de doxorubicine ou d'épirubicine lié à un polymère.

La présente invention vise précisément à préparer un nouveau mode de formulation d'actifs thérapeutiques faiblement hydrosolubles permettant de suppléer aux inconvénients des formulations conventionnelles de tels actifs.

La présente invention résulte plus particulièrement de l'observation par les inventeurs qu'il s'avère possible de formuler ces actifs thérapeutiques, faiblement hydrosolubles voire hydrophobes, à l'état de nanoparticules en suspension dans un milieu aqueux et de taille réduite, notamment compatible pour une administration par voie injectable, sous réserve d'associer ces actifs à un dérivé squalénique..

Ainsi, la présente invention concerne selon un premier aspect, un dérivé hydrodispersible d'un actif thérapeutique de faible solubilité aqueuse choisi parmi les taxoïdes, la doxorubicine et l'épirubicine ledit dérivé étant formé d'au moins une molécule dudit actif couplée de manière covalente à au moins une molécule d'un composé hydrocarboné à structure squalénique .

Plus précisément, la présente invention concerne un dérivé hydrodispersible précité dans lequel l'actif thérapeutique mis en œuvre possède une solubilité inférieure à 100 µg/ml dans de l'eau pure mesurée à température ambiante, en particulier inférieure à 25 µg/ml, notamment inférieure à 20 µg/ml, voire inférieure à 10 µg/ml et plus particulièrement inférieure à 5 µg/ml.

Au sens de la présente invention, un tel dérivé peut également être nommé « conjugué ».

Plus particulièrement, la présente invention concerne, selon un autre de ses aspects, des nanoparticules hydrodispersibles d'au moins un actif thérapeutique de faible solubilité aqueuse choisi parmi les taxoïdes, la doxorubicine et l'épirubicine dans lesquelles ledit actif y est présent sous une forme associée à au moins un composé hydrocarboné à structure squalénique .

Dans le document PCT/FR2005/050488, les inventeurs ont déjà fait état de l'aptitude du squalène, lorsqu'il est couplé de manière covalente à la gemcitabine, une molécule hydrophile et polaire, à former spontanément des nanoparticules d'une centaine de nanomètres en milieu aqueux. Cette capacité y est notamment expliquée par le comportement amphiphile des dérivés ainsi synthétisés, la partie squalène représentant la partie hydrophobe et la partie gemcitabine la partie hydrophile.

Or, contre toute attente, les inventeurs ont constaté que la mise en présence de dérivés d'actifs thérapeutiques de faible solubilité aqueuse voire hydrophobes, résultant du couplage covalent d'au moins une molécule dudit actif thérapeutique avec au moins une molécule d'un composé hydrocarboné à squelette squalénique, avec un solvant polaire à l'image de l'eau par exemple, conduit également à la formation spontanée de particules de quelques dizaines à quelques centaines de nanomètres, donc avantageusement compatibles avec une administration par voie systémique et demeurant dotées de l'efficacité thérapeutique de l'actif considéré.

La formulation de ces actifs thérapeutiques hydrophobes à l'état de nanoparticules conformes à la présente invention constitue donc une alternative avantageuse au regard des formulations déjà existantes.

### Dérivé hydrocarboné à structure squalénique

Au sens de la présente invention, une « structure squalénique » entend désigner une structure hydrocarbonée, linéaire, formée d'unités isoprène, et plus particulièrement au nombre de 6, à l'image du squalène dont la formule est la suivante :

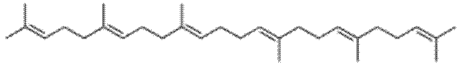

A titre illustratif de ces composés hydrocarbonés, on peut plus particulièrement citer l'acide squalénique et ses dérivés.

Comme les inventeurs l'ont constaté, cette structure squalénique est particulièrement importante dans le cadre de la présente invention car elle manifeste spontanément, lorsqu'elle est mise en présence d'un milieu polaire et plus particulièrement l'eau, une conformation compactée.

De manière inattendue, les inventeurs ont constaté que cette aptitude demeure lorsqu'un tel dérivé est associé et notamment lié de manière covalente à une autre entité chimique également de caractère hydrophobe. Il s'en suit la génération d'une architecture compactée à l'état de nanoparticules dans laquelle les deux entités chimiques sont intimement imbriquées l'une dans l'autre.

Au sens de la présente invention, sont notamment couverts sous cette définition les formes substituées des dérivés du squalène et en particulier l'acide squalénique et ses dérivés, notamment de substitution.

Un tel dérivé peut être par exemple à l'image de l'acide 1, l', 2-tris-norsqualénique, l'acide squalénoyl-acétique, la 1, l', 2-tris-norsqualénamine, le 1, l', 2-tris-norsqualénol, le 1, l', 2-tris-norsqualénethiol, l'acide squalénacétique, le squalényléthanol, le squalényléthanethiol ou encore la squalényléthylamine

Généralement au moins une molécule hydrocarbonée à structure squalénique est liée de manière covalente à une molécule d'un actif thérapeutique de faible solubilité aqueuse. Bien entendu, le nombre de molécules de dérivé hydrocarboné susceptible d'interagir avec une molécule d'actif thérapeutique peut être supérieur à 1. Un dérivé selon l'invention peut comprendre au moins deux radicaux à structure squalénique, identiques ou différents.

Ce composé hydrocarboné est généralement porteur d'une fonction susceptible de réagir avec une fonction présente sur la molécule de l'actif considérée de manière à établir un lien covalent entre les deux entités par exemple de type ester, éther, thioéther, disulfure, phosphate ou amide. Avantageusement, il s'agit d'une fonction carboxylique. Auquel cas, le dérivé hydrocarboné à structure squalénique est l'acide squalénique ou l'un de ses dérivés tels que par exemple l'ester de squalénoyl N-hydroxysuccinimidyle.

Selon une variante de réalisation, le lien covalent existant entre les deux types de molécules peut être figuré par un « linker » ou encore bras de liaison. Un tel bras peut notamment s'avérer utile lorsque les fonctions respectivement présentes sur le composé à structure squalénique et l'actif thérapeutique de faible solubilité n'ont pas d'affinité réactionnelle l'une vis-à-vis de l'autre et donc ne sont pas susceptibles de former le lien covalent attendu. Un tel bras permet précisément d'introduire *via* chacune des deux extrémités de son squelette les fonctions adéquates, i.e. possédant respectivement l'affinité réactionnelle attendue, l'une pour la fonction présente sur le dérivé à structure squalénique et l'autre pour la fonction présente sur l'actif considéré.

On peut également envisager que ce bras de liaison possède en outre au niveau de son squelette une fonction labile, propice ultérieurement à la séparation du composé à structure squalénique de l'actif thérapeutique. Il peut par exemple s'agir d'un motif peptidique reconnaissable par une enzyme.

Les motifs de type bras de liaison sont bien connus de l'homme de l'art et leur mise en œuvre relève clairement de ses compétences.

A titre représentatif des bras de liaison envisageables selon l'invention, on peut notamment citer les motifs (poly)aminoacides, polyols, saccharidiques, et polyéthylèneglycol (polyétheroxides) de faible poids moléculaire, en particulier polyols, saccharidiques et polyéthylèneglycol (polyétheroxides) de faible poids moléculaire.

Ainsi, au sens de la présente invention, un « lien covalent » figure de préférence une liaison covalente notamment telle que précisée ci-dessus, mais couvre également un lien covalent figuré par un bras de liaison tel que défini précédemment.

### Actifs thérapeutiques de faible solubilité

Au sens de la présente invention, un actif thérapeutique de faible solubilité aqueuse est un composé possédant une solubilité inférieure à 100 µg/ml dans de l'eau pure, mesurée à température ambiante c'est-à-dire 25 °C environ, notamment inférieure à 25 µg/ml, en particulier inférieure à 20 µg/ml, notamment inférieure à 15 µg/ml voire inférieure à 10 µg/ml et plus particulièrement inférieure à 5 µg/ml. Au sens de l'invention, une eau pure est une eau de pH proche de la neutralité (entre pH5 et pH8) et dénuée de tout autre composé tel que des sels organiques ou inorganiques par exemple.

A titre représentatif de ces substances de faible solubilité aqueuse, peuvent être notamment cités les immunosuppresseurs, les agents chimiothérapeutiques, notamment antitumoraux, comme les taxoïdes, la doxorubicine encore appelée adriamycine et son isomère l'épirubicine, les agents antiangiogéniques, des antiviraux, antibactériens, antibiotiques et antiparasitaires, les substances agissant sur le métabolisme des sucres, les peptides, les lipides, les agents agissant sur les canaux calciques, les antiflogistiques non stéroidiens et les composés peptidiques comme l'insuline.

Il est entendu que dans le cadre de la présente invention ce sont uniquement les formes hydrophobes ou de très faible solubilité des taxoïdes, de la doxorubicine encore appelée adriamycine et de son isomère l'épirubicine, qui sont considérées. Leurs formes ioniques ou autres, susceptibles d'êtres solubles en milieu aqueux ne présentant pas d'intérêt dans le cadre de l'invention dans la mesure où il ne s'avère pas nécessaire de suppléer à leur égard, à un défaut de solubilité aqueuse.

Ainsi, dans le cas de la présente invention, la forme de l'actif couplée à au moins une molécule à structure squalénique est généralement une forme neutre, c'est-à-dire non ionique ou non salifiée sauf si celle-ci s'avère elle-même de faible hydrosolubilité.

Les immunosuppresseurs qui ne font pas partie de l'invention sont des composés hydrophobes et comprennent les undécapeptides cycliques N-méthylés. Parmi cette famille d'actifs, les cyclosporines sont plus particulièrement considérées. Il s'agit notamment des cyclosporines A et G. Toutefois, d'autres macrolides peuvent également être considérés.

Il peut également s'agir d'un immunosuppresseur tel que le déoxyspergualine, le rapamycine ou l'ascomycine.

Selon une variante préférée de l'invention, les actifs thérapeutiques de faible solubilité sont plus particulièrement les taxanes et les taxoïdes.

De tels composés sont notamment décrits dans la demande WO 2005/013968. Il s'agit plus préférablement du docétaxel, paclitaxel ou un de leurs dérivés.

Comme précisé précédemment, ces substances sont fonctionnalisées avec au moins une molécule d'un composé hydrocarboné à structure squalénique. Ainsi, les actifs thérapeutiques considérés selon la présente invention peuvent comprendre deux dérivatisations, trois dérivatisations, voire plus, celles-ci pouvant être identiques ou différentes.

La réaction nécessaire à l'établissement d'au moins une liaison covalente entre une molécule d'un actif thérapeutique de faible solubilité aqueuse et au moins une molécule d'un dérivé hydrocarboné de structure squalénique peut être effectuée selon des conditions standard et sa réalisation relève donc clairement des connaissances de l'homme de l'art.

Cette réaction est généralement réalisée en solution en présence et en excès d'au moins un composé à structure squalénique par rapport à l'actif faiblement hydrosoluble considéré, par exemple à raison de deux équivalents, selon les conditions standard requises pour faire interagir les deux fonctions spécifiques portées par chacune des molécules.

A titre illustratif des dérivés hydrodispersibles conformes à la présente invention, peuvent tout particulièrement être cités les dérivés taxoïdes suivants ;

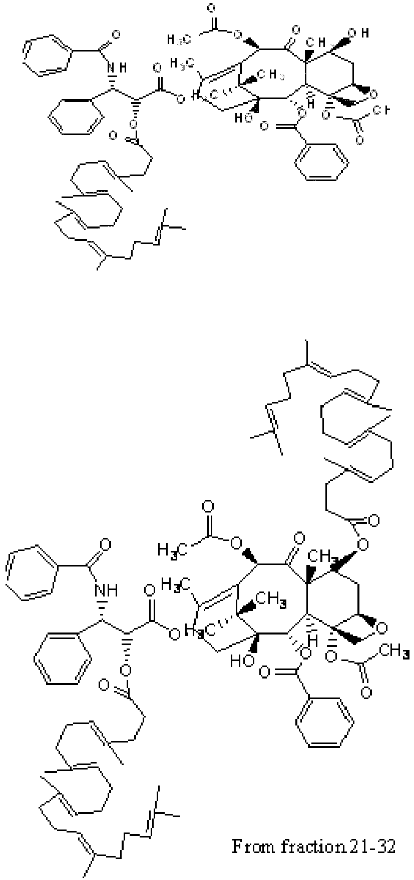

Il s'agit de deux composés dérivant de la fonctionnalisation d'une molécule de paclitaxel avec respectivement une et deux molécules d'acide squalénique.

D'autres dérivés hydrodispersibles sont :
- le squalénoyl-insuline de formule comme suit qui ne fait pas partie de l'invention : 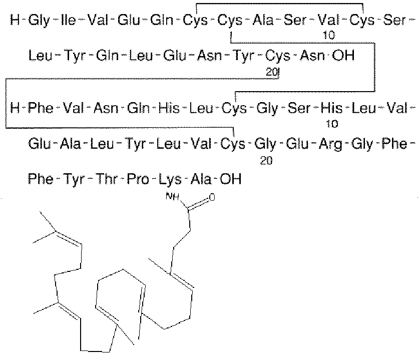
- le squalénoyl-épirubicine de formule comme suit conforme à l'invention: 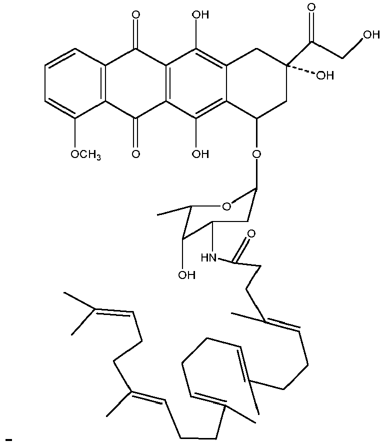
- et le squalénoyl-désoxyspergualine de formule comme suit qui ne fait pas partie de l'invention :

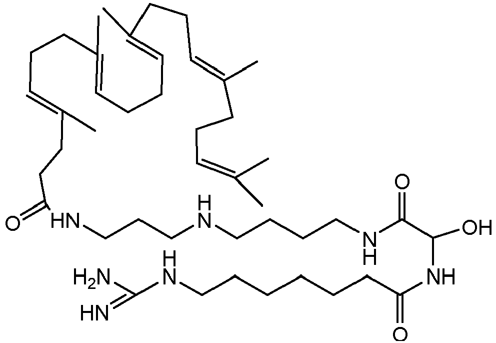

Ces composés sont généralement obtenus sous la forme d'une dispersion aqueuse.

Ainsi, selon un autre de ses aspects, la présente invention concerne une dispersion aqueuse d'au moins un dérivé tel que défini précédemment et faisant partie de l'invention.

### Nanoparticules selon l'invention

Comme précisé précédemment, le couplage covalent d'un actif thérapeutique avec au moins une molécule d'un composé hydrocarboné à structure squalénique est de nature à conférer à l'actif ainsi fonctionnalisé avec au moins un radical squalénoyle une aptitude à s'organiser sous une forme compactée dans un milieu solvant polaire, conduisant ainsi à la formation de nanoparticules.

D'une manière générale, les nanoparticules ainsi obtenues possèdent une taille moyenne variant de 30 à 650 nm, en particulier de 30 à 500 nm, et en particulier de 50 à 250 nm, voire de 100 à 200 nm mesurée par diffusion de la lumière à l'aide du nanosizer Coulter® N4MD, Coulter Electronics, Hialeah, USA.

Ainsi, l'interaction d'un actif thérapeutique faiblement hydrosoluble comme considéré selon l'invention avec un dérivé hydrocarboné conforme à l'invention, et plus particulièrement avec l'acide squalénique ou l'un de ses dérivés comme par exemple, l'ester de squalénoyl N-hydroxysuccinimidyle, confère à ladite substance thérapeutique des caractéristiques physico-chimiques suffisantes pour lui conférer une aptitude à former des particules dont la taille s'avère compatible pour une administration parentérale et notamment par voie intraveineuse.

La présente invention concerne selon un autre de ses aspects, un procédé de préparation de ces nanoparticules, caractérisé en ce qu'il comprend :
- la solubilisation d'un dérivé selon l'invention c'est-à-dire formé préliminairement par couplage d'au moins une molécule d'un composé hydrocarboné à structure squalénique à une molécule d'un actif thérapeutique de faible solubilité aqueuse étant un agent antitumoral chimiothérapeutique choisi parmi les taxoides, la doxorubicine et l'épirubicine, dans au moins un solvant organique, par exemple un alcool comme l'éthanol, à une concentration suffisante pour obtenir, lors de l'ajout du mélange résultant, sous agitation, et généralement au goutte-à-goutte, à une phase aqueuse, la formation instantanée de nanoparticules dudit dérivé en suspension dans ladite phase aqueuse, et,
- le cas échéant, l'isolement desdites nanoparticules.

La réaction peut généralement être réalisée à température ambiante. Quelle qu'elle soit, la température de réaction ne doit pas affecter l'activité de l'actif considéré. Le procédé de préparation des nanoparticules selon l'invention est particulièrement avantageux dans la mesure où il ne requiert pas la présence de tensioactifs.

Comme précisé précédemment, le couplage entre le dérivé hydrocarboné à structure squalénique et la molécule d'actif peut être direct ou *via* un bras de liaison.

Les inventeurs ont en outre constaté qu'il était possible de contrôler la taille de ces particules à travers la quantité de l'actif thérapeutique mis en œuvre pour la nanoprécipitation. En effet, l'augmentation de la concentration en produit de couplage entraîne généralement une augmentation de la taille, et inversement.

Selon un mode de réalisation avantageux, les nanoparticules selon l'invention sont formulées à l'état de dispersion aqueuse en vue de leur administration généralement par voie systémique.

Selon un mode de réalisation avantageux, cette dispersion aqueuse contient moins de 5 % en poids, voire moins de 2 % en poids en alcool en C₂ à C₄ tel que par exemple l'éthanol.

Selon un autre mode de réalisation avantageux, cette dispersion aqueuse contient moins de 5 % en poids, voire moins de 2 % en poids et plus particulièrement est dénuée de tensioactif ou analogue tels que par exemple les polyéthylène glycols, le polyglycérol et leurs dérivés, tels les esters par exemple.

Selon un autre mode de réalisation avantageux, cette dispersion aqueuse contient moins de 5 % en poids, voire moins de 2 % en poids et plus particulièrement est dénuée d'huile de ricin polyoxyéthylée telle que par exemple celle commercialisée sous la dénomination Crémophore EL®.

Selon un autre mode de réalisation avantageux, cette dispersion aqueuse possède intrinsèquement une viscosité compatible avec une administration par voie intraveineuse.

Ainsi, la formulation en milieu aqueux du taxoïde tel que le Paclitaxel® à l'aide de l'acide squalénique à l'état de nanoparticules hydrodispersibles permet, avantageusement, d'obtenir une suspension de nanoparticules sans autre additif que le dextrose 5 % nécessaire pour obtenir l'isotonie de la suspension injectable. Il s'avère ainsi possible de : (i) s'affranchir de l'utilisation du Crémophore toxique, (ii) disposer d'une formulation aqueuse directement injectable et (iii) administrer des concentrations plus importantes du produit (jusqu'à 4 mg/ml).

La présente invention concerne également selon un autre de ses aspects, l'utilisation de ces dérivés et nanoparticules dans des compositions pharmaceutiques.

Elle concerne en outre une composition pharmaceutique comprenant, à titre de matière active, au moins un dérivé conforme à la présente invention, notamment sous la forme de nanoparticules.

Les dérivés conformes à la présente invention peuvent également être administrés par toutes les voies conventionnelles. Toutefois, comme précisé précédemment, compte tenu de la faible taille de leurs particules, ils sont administrables sous la forme d'une suspension aqueuse par voie intraveineuse et donc compatibles avec la microcirculation vasculaire.

Un autre aspect de l'invention concerne donc une composition pharmaceutique comprenant au moins, au titre de matière active, un composé conforme à la présente invention notamment sous la forme de nanoparticules. Les dérivés conformes à la présente invention peuvent y être associés avec au moins un véhicule pharmaceutiquement acceptable.

A titre d'exemples de formulations pharmaceutiques compatibles avec les compositions selon l'invention, on peut notamment citer :
- les injections ou perfusions intraveineuses ;
- les solutions salines ou d'eau purifiée ;
- les compositions pour inhalation ;
- les capsules, dragées et cachets incorporant notamment à titre de véhicule, de l'eau, du phosphate de calcium, des sucres, tels que lactose, dextrose ou mannitol, du talc, de l'acide stéarique, de l'amidon, du bicarbonate de sodium et/ou de la gélatine.

Lorsque les composés sont utilisés en dispersion dans une solution aqueuse, ils peuvent être associés à des excipients de type agent séquestrant ou chélatant, antioxydant, agents modifiant le pH et/ou agents tampons.

Les nanoparticules selon l'invention sont bien entendu susceptibles de porter en surface une multitude de fonctions réactives, à l'image des fonctions hydroxyle ou amine par exemple. Il est donc envisageable de fixer à ces fonctions toutes sortes de molécules, notamment par des liaisons covalentes.

A titre illustratif et non limitatif de ce type de molécules susceptibles d'être associées aux nanoparticules, on peut notamment citer les molécules de type marqueur, les composés susceptibles d'assurer une fonction de ciblage, ainsi que tout composé apte à leur conférer des caractéristiques pharmacocinétiques particulières. En ce qui concerne ce dernier aspect, on peut ainsi envisager de fixer en surface de ces nanoparticules des dérivés lipophiles du polyéthylène glycol, comme par exemple le polyéthylène glycol cholestérol ou le polyéthylène glycol-phosphatidyléthanolamine, ou mieux encore le polyéthylène glycol squalène. Un enrobage de surface à base d'un tel composé est en effet avantageux pour conférer une rémanence vasculaire accrue en raison d'une réduction significative de la capture des nanoparticules par les macrophages hépatiques.

Outre les composés précités, les compositions pharmaceutiques selon l'invention peuvent contenir des agents de type conservateurs, des agents mouillants, des agents solubilisants et des agents de coloration.

Pour des raisons évidentes, les quantités en dérivés selon l'invention susceptibles d'être mis en œuvre sont susceptibles de varier significativement selon le mode d'utilisation et la voie retenue pour leur administration.

Par exemple, pour un traitement par voie systémique en taxoïde, destiné à un patient de type adulte, on peut envisager d'administrer un dérivé conforme à la présente invention à une dose d'environ 0,1 à 150 mg/kg de poids corporel et par jour et plus particulièrement de 1 à 40 mg/kg par jour.

En revanche, pour une administration topique on peut envisager de formuler au moins un dérivé conforme à la présente invention à raison de 0,1 à 40 % en poids, voire plus, par rapport au poids total de la formulation pharmaceutique considérée.

Il est également possible de co-administrer au moins un dérivé conforme à la présente invention avec au moins une autre matière active susceptible d'être également bénéfique à l'égard de la pathologie considérée.

A titre représentatif de ces matières actives susceptibles d'être combinées aux dérivés conformes à la présente invention, on peut notamment citer d'autres molécules ou macromolécules anticancéreuses ou cytostatiques (par exemple sels de platine, antracyclines, poisons du fuseau mitotique, inhibiteurs de topoisomérases, de kinases ou de métalloprotéases), des agents anti-inflammatoires de type corticoïde (par exemple dexaméthasone) ou non corticoïde ou encore des molécules à activité immunoadjuvante (par exemple anticorps à activité anticancéreuse). L'association avec l'hyperthermie utilisée dans certaines chimiothérapies peut être envisagée. Les dérivés conformes à la présente invention peuvent également être combinés aux thérapies chirurgicales et/ou aux radiations pour le traitement du cancer.

Les exemples et figures, figurant ci-après, sont présentés à titre illustratif du domaine de l'invention.
Figure 1 : Accumulation de la tubuline après incubation des cellules KB avec les nanoparticules de squalénoylpaclitaxel (1B) au regard des cellules KB non traitées (1A).
Figure 2 : Evaluation de l'activité anti-cancer *in vivo* des nanoparticules d'un dérivé de squalénoyldiglycolyl-paclitaxel obtenu selon l'Exemple 11, par caractérisation du volume d'une tumeur implantée sur une souris (en mm³) en fonction du temps (nombre de jours après implantation de la tumeur).
Figure 3 : Evaluation des variations du poids de souris porteuses de leucémie L1210, traitées ou non avec les nanoparticules de squalénoyl-doxorubicine, induites par les métastases, en fonction du temps (nombre de jours après injection intra-veineuse de cellules leucémique L1210).
Figure 4 : Evaluation du taux de survie des souris porteuses de leucémie L1210, traitées ou non avec les nanoparticules de squalénoyl-doxorubicine, en fonction du temps (nombre de jours après injection intra-veineuse de cellules leucémique L1210).

### Exemple 1

### Synthèse du monosqualénoylpaclitaxel

450 mg de Paclitaxel (0,526 mmol) dans du dichlorométhane (DCM) sont mis à réagir avec du 1-ethyl-3-(3-diméthylamino-propyl carbodiimide (EDCA, 2,5 équivalents molaires par rapport au Paclitaxel), du diméthylamino pyridine (DMAP, 0,5 équivalent molaire par rapport au Paclitaxel) et de l'acide squalénique (2 équivalents molaires par rapport au Paclitaxel) préalablement dissous dans du DCM à température ambiante. Après 1 h, la réaction est terminée et stoppée à l'aide d'eau à pH 5 et l'extraction est réalisée avec une solution aqueuse de chlorure de sodium (NaCl). La phase aqueuse est ensuite lavée avec le DCM. La purification du mélange obtenu se fait par chromatographie Flash SiO₂ (2,5 x 40 cm) éluée avec un mélange de DCM/acétate d'éthyle. Les fractions 62-80 sont récoltées et le monosqualénoylpaclitaxel ainsi obtenu est caractérisé par RMN ¹H (Bruker 300 Mhz), spectroscopie de masse (Micromass Waters ESI) et RP-HPLC.

Formule chimique : C₇₄H₉₃NO₁₅ ; PM : 1236,53

### Exemple 2

### Synthèse du disqualénoylpaclitaxel

450 mg de Paclitaxel (0,526 mmol) dans du dichlorométhane (DCM) sont mis à réagir avec du 1-éthyl-3-(3-diméthylamino-propyl)-carbodiimide (EDCA, 2,5 équivalents molaires par rapport au Paclitaxel), du diméthylamino pyridine (DMAP, 0,5 équivalent molaire par rapport au Paclitaxel) et de l'acide squalénique (2 équivalents molaires par rapport au Paclitaxel) préalablement dissous dans du DCM à température ambiante. Après 1 h, la réaction est terminée et stoppée à l'aide d'eau à pH 5 et l'extraction est réalisée avec une solution aqueuse de chlorure de sodium (NaCl). La phase aqueuse est ensuite lavée avec le DCM. La purification du mélange obtenu se fait par chromatographie Flash SiO₂ (2.5 x 40 cm) éluée avec un mélange de DCM/acétate d'éthyle. Les fractions 21-32 sont récoltées et le disqualénoylpaclitaxel ainsi obtenu est caractérisé par RMN ¹H (Bruker 300 Mhz), spectroscopie de masse (Micromass Waters ESI) et RP-HPLC.

Formule chimique : C₁₀₁H₁₃₅NO₁₆ ; PM : 1619,15

### Exemple 3

### Préparation des nanoparticules de squalénoylpaclitaxel

4 mg de squalénoylpaclitaxel sont dissous dans 1,5 ml d'éthanol (2,5 mg/ml) et ajoutés goutte à goutte et sous agitation continue (vitesse 500 tpm) à 1 ml d'une solution aqueuse contenant 5 % de dextrose. Le squalénoylpaclitaxel s'auto-assemble sous forme de nanoparticules d'une taille comprise entre 100 et 200 nm. L'éthanol est ensuite complètement évaporé sous pression réduite à l'aide d'un rotavapor® pour obtenir une suspension nanoparticulaire de squalénoylpaclitaxel à la concentration finale de 4 mg/ml.

### Exemple 4

### Préparation d'autres nanoparticules de squalénoylpaclitaxel

Des quantités variables (précisées en tableau 1 ci-après) de squalénoylpaclitaxel sont dissoutes dans 0,25 ml de tétrahydrofurane (THF). Les solutions obtenues sont ajoutées goutte à goutte et sous agitation continue (vitesse 500 tpm) à 0,25 ml d'eau. Le squalénoylpaclitaxel précipite sous forme de nanoparticules d'une taille comprise entre 100 et 200 nm. Le THF est ensuite complètement évaporé sous pression réduite à l'aide d'un rotavapor® pour obtenir des suspensions nanoparticulaires de squalénoylpaclitaxel dont la taille varie en fonction de la concentration (voir tableau ci-dessous)

**Tableau 1**

| Squalénoyl paclitaxel (mmol) | Diamètre (nm) | Index de Polydispersité |
|---|---|---|
| 0,36 | 151 ± 84 | 0,39 |
| 0,88 | 169 ± 92 | 0,30 |
| 1,76 | 177 ± 99 | 0,31 |

### Exemple 5

### Injection des nanoparticules de squalénoylpaclitaxel aux souris et comparaison avec le TAXOL

La suspension nanoparticulaire de squalénoylpaclitaxel telle que préparée à l'exemple 3 (4 mg/ml) est directement injectée par voie intraveineuse à des souris (C57BL6) à la dose de 4 x 50 mg/kg (équivalent Paclitaxel) ; les injections ont lieu aux jours 0, 5, 9 et 14. L'injection est aisée et les animaux ne présentent aucun signe de toxicité. Aucune mortalité n'est enregistrée (dans un délai d'au moins un mois). L'injection intraveineuse directe de la solution de TAXOL (sans dilution dans du NaCl 0,9 %) conduit à la mort immédiate de 100 % des souris traitées. Lorsque la solution de TAXOL est préalablement diluée dans du NaCl 0,9 %, la dose maximale qui peut-être injectée est de 20 mg/kg.

### Exemple 6

### Activité anticancéreuse des nanoparticules de squalénoylpaclitaxel

La caractérisation de cette activité est réalisée par immunofluorescence selon le protocole suivant :
Jour 1 : les cellules KB 3,1 provenant d'un carcinome cervical humain, et les cellules HT-29 correspondant à une lignée humaine de cellules cancéreuses du colon, sont déposées sur des plaques à 6 puits à une densité de 20000 cellules/puits (volume final 2 ml RPMI plus FCS). Les cellules sont incubées à une température de 37 °C et à 5 % de CO₂.
Jour 2 : Les cellules sont rincées et on y ajoute les nanoparticules de squalénoyl paclitaxel (concentration 5 µM) tel que préparées selon le protocole décrit dans l'exemple 3.
Jour 3 : Après 14-16 h, on rince deux fois avec du PBS puis on ajoute la solution d'extraction (Triton X-100 plus 0,5 % PEM - PIPES 100 mM, EGTA 2 mM , MgCl₂ 2 mM pH 6.8). Après 4 min, on rince puis on ajoute 2 ml d'une solution à 3 % de formaldéhyde dans le PEM. 40 min plus tard, on rince puis on ajoute 20 % de FCS dans le PBS pendant 30 min. On rajoute ensuite l'anticorps monoclonal murin anti-α-tubulin-FITC (dilué à 1:200) et on laisse incuber pendant 1 h. On rince enfin avec du PBS et on procède à une analyse par microscopie confocale de la fluorescence (Leica TCS SP2).

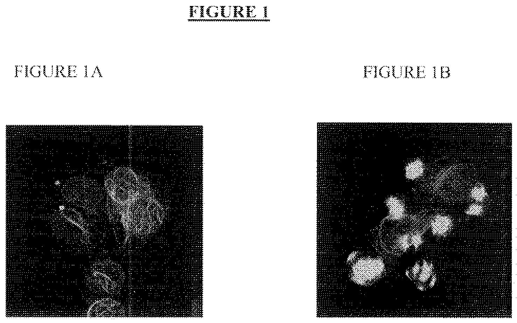

Comme illustrée par la figure 1B, la formation d'amas de tubuline est clairement visible en microscopie à fluorescence, ce qui montre l'activité de poison du fuseau des nanoparticules de squalénoylpaclitaxel. Les cellules KB non traitées sont présentées à titre de contrôle (figure 1A).

### Exemple 7 hors invention

### Préparation de nanoparticules de squalénoyl-insuline

### a) Obtention du squalénoyl N-hydroxysuccinimidyle

La réaction avec l'insuline requiert au préalable la préparation et l'isolation de l'ester de squalénoyl N-hydroxysuccinimidyle, un intermédiaire réactif. Pratiquement, 300 mg (0,75 mmol) d'acide squalénique dissous dans 2 ml de dichlorométhane (DCM) sont mis à réagir avec 72,5 mg de N-hydroxysuccinimide (2,0 excès molaire) et 309 mg dicyclohexylcarbodiimide (DCCD) (2x) dissous dans du dichlorométhane et maintenus sous agitation pendant 2 h. On enlève ensuite l'urée précipitée, puis on dissout le mélange dans de l'acétate d'éthyle, on le filtre et on le conserve à une température de - 20 °C. Les esters de squalénoyl N-hydroxysuccinimidyle (Sq-CO-NHS) sont caractérisés par RMN et par spectrométrie de masse.

### b) Obtention du conjugué de squalénoyl-insuline

50 mg d'insuline (origine pancréas bovin et répondant aux critères des tests USP) sont dissous dans 5 ml de diméthylsulfoxide sec (> 99 % de pureté) et ajoutés à 30 µl de N,N-diisopropyléthylamine dans un flacon contenant 13,2 mg de Sq-CO-NHS (dans un excès molaire de 3:1). La réaction est effectuée à température ambiante pendant 2 h et la solution est intensivement dialysée afin d'enlever les solvants organiques et les petites impuretés moléculaires, puis lyophilisée. Le solide est ensuite lavé avec du DCM puis filtré. On obtient 46 mg de poudre blanche.

La réaction de conjugaison est contrôlée par HPLC analytique en utilisant une colonne Waters RP C18 (150,9 mm, 5 µm, 300 Å) et du méthanol/eau (gradient plus 0,1 % TFA) comme solvant d'élution

La caractérisation du conjugué squaléné de l'insuline a été réalisée par des analyses MALDI-TOF et Orbi-Trap MS. Le composé répond à la formule développée suivante :

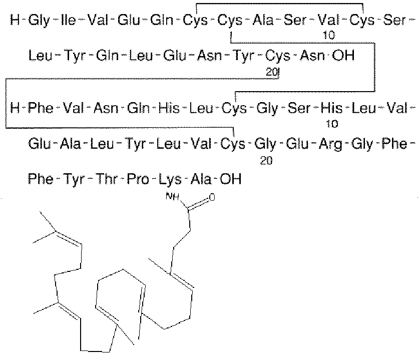

### c) Préparation des nanoparticules de squalénoyl-insuline.

4 mg du conjugué squalène de l'insuline purifié sont dissous dans du diméthylsulfoxide (DMSO) sec (1 ml) et dialysés de manière intensive avec de l'eau distillée. Les nanoparticules se forment durant le processus de dialyse. Le volume final est réduit à 1 ml par concentration solide polyéthylène glycol (PEG) 10 kDa.

### Exemple 8

### Préparation de nanoparticules de squalénoyl-épirubicine

### a) Obtention du dérivé 3' amido squalénoyl-épirubicine

On dissout rapidement 100 mg d'épirubicine dans 2 ml de diméthylformamide (DMF) sec que l'on mélange à une solution de 180 mg d'ester de squalénoyl N-hydroxysuccinimide (excès molaire : 2,0) préalablement dissous dans du DMF sec et on ajoute 20 µl de N,N-diisopropyléthylamine. Après mélange pendant 2 h à une température de 25 °C, on dilue la solution avec du DCM et on lave avec de l'eau distillée. On réalise la purification à l'aide d'une chromatographie flash sur SiO₂ (2,5 x 25 cm) éluée avec un mélange DCM-éthanol. Les fractions 60-67 sont récoltées et 100 mg du conjugué 3'amido squalénoyl-épirubicine sont obtenus et caractérisés par RMN et spectrométrie de masse. Le composé répond à la formule développée suivante :

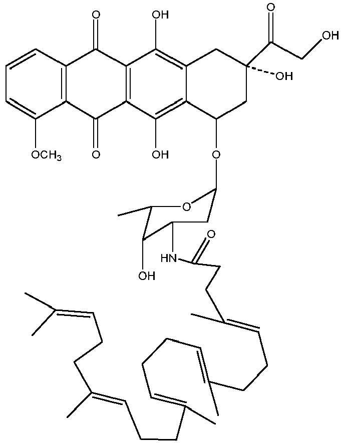

### b) Préparation de nanoparticules de squalénoyl-épirubicine

Un volume de 200 µl de squalenoyl-3-épirubicine dissous dans l'acétone (10 mg/ml) est ajouté au goutte-à-goutte à 5 ml d'eau distillée, sous forte agitation. Une fine suspension rouge est immédiatement obtenue. Après évaporation de l'acétone sous pression réduite à l'aide d'un rotavapor®, on obtient une suspension nanoparticulaire stable.

### Exemple 9 hors invention

### Préparation de nanoparticules de squalénoyl-déoxyspergualine

### a) Obtention de la squalénoyl-déoxyspergualine

On prépare d'abord la déoxyspergualine par purification du produit commercial dénommé Gusperimus (Spanidine). Après deux processus chromatographiques (échange ionique et filtration sur gel), on met en réaction la forme basique de la déoxyspergualine (faiblement soluble dans l'eau) avec l'ester de squalénoyl N-hydroxysuccinimidyle. Pour ce faire, on dissout rapidement 24 mg de déoxyspergualine dans 1 ml de DMF sec que l'on ajoute à 29 mg d'ester de squalénoyl N-hydroxysuccinimide (excès molaire : 1,3) préalablement dissous dans le même solvant. On ajoute ensuite 10 µl de N,N-diisopropylethylamine. Après mélange pendant 2 h à une température de 25 °C, on dilue la solution avec du DCM et on lave avec de l'eau distillée. On réalise la purification *via* une chromatographie flash sur SiO₂ (1,5 x 28 cm) éluée avec du DCM/éthanol plus 1 % de triéthylamine (TEA). On récolte ensuite les fractions qui se sont révélées positives au test de Sakaguchi (groupe guanidine). 15 mg du conjugué de squalénoyl-déoxyspergualine sont obtenus puis caractérisés par RMN et spectrométrie de masse. Le conjugué de squalénoyl-déoxyspergualine a la formule développée suivante :

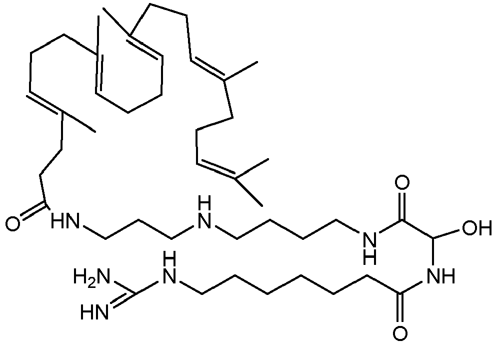

### b) Préparation des nanoparticules de squalénoyl-déoxyspergualine

100 µl de squalénoyl-déoxyspergualine dissous dans de l'éthanol (4 mg/ml) sont ajoutés au goutte-à-goutte à 2 ml d'eau distillée sous forte agitation. Après évaporation de l'éthanol sous pression réduite à l'aide d'un rotavapor®, on obtient une suspension de nanoparticules d'une taille de 162 nm (indice de polydispersité 0,12 caractérisé par un potentiel de surface de 27,38 +/- 2,60 mV).

### Exemple 10

### Synthèse du squalénoylsuccinyl-paclitaxel

### Etape 1

Le paclitaxel est dissous dans de la 4-diméthylaminopyridine (0,1 eq.) et de l'anhydride succinique (2 eq.) et séché sous vide durant 2 heures. Après ajout de pyridine sèche, le mélange réactif est agité pendant 3 heures à température ambiante. Après élimination du solvant, le mélange brut est dissous dans du dichlorométhane (DCM) et rincé avec de la saumure. Aucune étape de purification supplémentaire du paclitaxel-2'-succinate n'est nécessaire. L'analyse RMN pour détecter l'absence de 2' OH, la conversion de 2' C-H (à 5,51) et la présence quantitative de 7 C-H en position normale (4,48) montre la transformation totale du matériau de départ en dérivé succinate.

### Etape 2

Le N-hydroxysuccinimido-diphényle phosphate (SDPP) est préparé à partir de chlorure de diphénylphosphoryle, de N-hydroxysuccinimide, et de triéthylamine (TEA) dans du DCM. Le SDPP brut est trituré dans de l'éther, dissous dans de l'acétate d'éthyle, rincé à l'eau, séché et concentré sous vide afin d'obtenir du SDPP. La caractérisation par spectrométrie de masse (MS) confirme un pic moléculaire à 348.

A une solution de paclitaxel-2'-succinate est ajouté du SDPP (1,5 eq.) dans de l'acétonitrile avec de la TEA (4 eq.). Le mélange réactionnel est agité pendant 6 heures à température ambiante, puis concentré sous vide. La réaction brute est dissoute dans de l'acétate d'éthyle et extraite à l'aide de saumure. La formation de paclitaxel-2'-succinyl-NHS est contrôlé par analyses TLC et HPLC, comme décrites ci-dessus, ce qui ne nécessite pas d'étape de purification supplémentaire.

### Etape 3

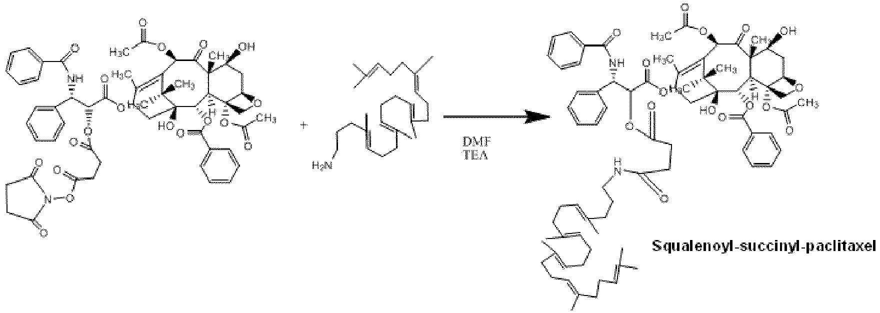

Le Paclitaxel-2'-succinyl-NHS dissous dans du diméthylformamide (DMF) sec est mis à réagir avec l'amine squalène (1 eq.) en présence de triéthylamine (1 eq.). Après 8 heures à température ambiante et une nuit à 5 °C, le mélange est extrait à l'aide de saumure et purifié sur un gel de SiO₂, élué avec de l'acétate de DCM-éthyle. Le produit principal (squalénoylsuccinyl-paclitaxel) est élué avec 30-50 % d'acétate d'éthyle et sa pureté est contrôlée par analyse HPLC sur une colonne RP-18.

¹H-NMR (CDCl₃): d 8,13 (d, 2H, OC(O) o-ArH), 7,75 (d, 2H, NC(O) o-ArH), 7,62 (t, 1H, OC(O) p-ArH), 7,53±7.49 (bande, 3H), 7,43±7.35 (bande, 7H), 6,91 (t, 1H, NH), 6,35 (s, 1H, C(10)-H), 6,27 (t, 1H, C(13)-H), 5,99 (t, 1H, C(3')-H), 5,69 (d, 1H, C(2)-H), 5,30 (dd,1H, C(7)-H), 5,44 (dd, 1H, C(2'-H), 5,20 (s, 5H, C(sq-H), 4,97 (d, 1H, C(5)-H) 4,39±4,25 (bande, 3H), 4,24±4,10 (bande, 7H), 3,96 (d,1H), 3,85 (dd, 1H), 3,75 (m, 1H), 3,42 (t, 2H, sq CH2-NH), 2,61 (p, 1H, C(6)-H et m 4H succinyle CH2), 2,46 (d, 3H, C(4)-OAc), 2,41 (m, 1H, C(14)-H), 2,29 et 2,20 (s 4H, CH2 sq. acide), 2,23(m, 1H, C(14)-H), 2,14 (d, 3H, C(10)-OAc), 2,01 (s, 3H, C(12)-CH3), 2,00 (m, 16H, CH2 sq), 1,97 (m, 1H, C(6)-H), 1,81 (s, 3H, C(8)-CH3), 1,71 (m, 18H, C(sq)-CH3), 1,41 (d, 3H), 1,21 (s,3H, C(15)-CH3), 1,16 (s, 3H, C(15)-CH3).

### Exemple 11

### Synthèse du squalénoyldiglycolyl-paclitaxel

### Etape 1

Le squaléne, sous forme alcoolique, est mélangé avec de l'anhydride diglycolique (2,5 eq) dans de la pyridine sèche à température ambiante pendant toute une nuit, sous agitation. Le solvant est éliminé et le résidu est extrait à partir d'acide chlorhydrique dilué et de la saumure avec du DCM. La conversion du produit attendu est contrôlée par TLC. Le produit ainsi obtenu est séché sous vide et l'acide est utilisé sans étape de purification supplémentaire.

### Etape 2

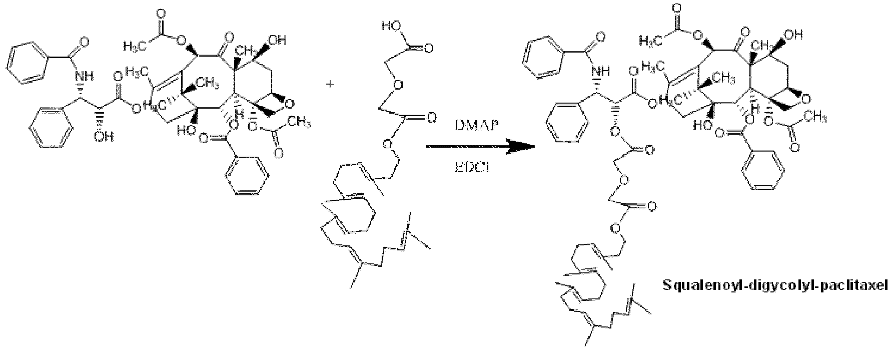

L'acide squalènediglycolique (2 eq.) décrit en étape 1 est dissous dans du DCM, puis le Paclitaxel (1 eq.) et la 4-N,N-dimethylaminopyridine (3 eq.), préalablement dissous dans du DCM, sont ajoutés. Après 10 minutes, l'EDCI (1,3 eq.) est ajouté et la solution est agitée à température ambiante pendant 2 heures. Après élimination du solvant, le produit brut est passé à travers une colonne de gel de silice en utilisant un gradient DCM/éthanol pour obtenir le produit purifié, dont la pureté est contrôlée par analyse HPLC sur une colonne RP-18.

¹H-NMR (CDCl3): d 8,13 (d, 2H, OC(O) o-ArH), 7,75 (d, 2H, NC(O) o-ArH), 7,62 (t, 1H, OC(O) p-ArH), 7,53±7,49 (bande, 3H), 7,43±7,35 (bande, 7H), 6,91 (t, 1H, NH), 6,35 (s, 1H, C(10)-H), 6,27 (t, 1H, C(13)-H), 5,99 (t, 1H, C(3')-H), 5,69 (d, 1H, C(2)-H), 5,30 (dd,1H, C(7)-H), 5,44 (dd, 1H, C(2'-H), 5,20 (s, 5H, C(sq-H), 4,97 (d, 1H, C(5)-H) 4,39±4,25 (bande, 3H), 4,33 (m, 4H diglycoyl CH2), 4,24±4,10 (bande, 7H), 4,12 (m, 2H C(1) squalène), 3,96 (d,1H), 3,85 (dd, 1H), 3,75 (m, 1H), 2,61 (p, 1H, C(6)-H),2,46 (d, 3H, C(4)-OAc), 2,41 (m, 1H, C(14)-H), 2,29 and 2,35 (s 4H, CH2 sq. acide ), 2,23(m, 1H, C(14)-H), 2,14 (d, 3H, C(10)-OAc), 2,01 (s, 3H, C(12)-CH3), 2,00 (m, 16H, CH2 sq), 1,97 (m, 1H, C(6)-H), 1,81 (s, 3H, C(8)-CH3), 1,71 (m, 18H, C(sq)-CH3), 1,41 (d, 3H), 1,21 (s,3H, C(15)-CH3), 1,16 (s, 3H, C(15)-CH3).

### Exemple 12

### Synthèse du squalènoylsuccinyl-PEG340-paclitaxel

### Etape 1

Le Paclitaxelsuccinyl-NHS (voir Exemple 10, étape 3) est dissous dans du DCM, et la solution de tBoc-NH-PEG3-NH₂ (1 eq.) dans du DCM est ajoutée à la solution précédente sous agitation. La triéthylamine (0,5 eq) est ajoutée à une température de 4 °C, et la réaction est maintenue à une température de 20 °C pendant 5 heures. Le mélange réactif brut est extrait avec 0,1N HCl, puis avec de la saumure. Le produit ainsi obtenu est utilisé à l'étape suivante sans purification supplémentaire.

### Etape 2

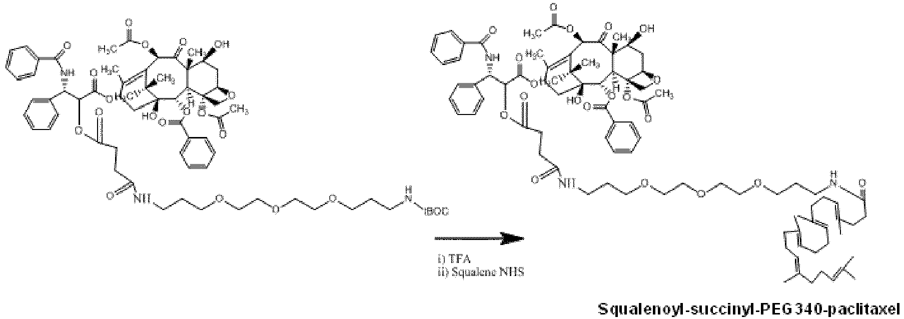

Le Paclitaxel-succinyl-Peg3-NH-tBoc est dissous dans du DCM sec, et de l'acide trifluoroacétique (TFA) est ajouté sous agitation à 20 °C. Après 2 heures, la réaction est terminée et le mélange est extrait avec une solution d'acétate de sodium, puis avec de la saumure jusqu'à la neutralité.

L'ester de Squalène N-hydroxysuccinimide (1.2 eq.), préalablement préparé par réaction d'acide squalénique et de NHS, en présence d'EDCI, est ajouté à la Paclitaxel-succinyl-Peg3-amine (1 eq.). De la triéthylamine (0.3 eq.) est ajoutée et le mélange réactif est agité, à température ambiante, pendant 6 heures. Après élimination du solvant, le produit brut est purifié au travers d'une colonne de gel de silice en utilisant un gradient DCM/éthanol afin d'obtenir le produit purifié, dont la pureté est contrôlée par analyse HPLC sur une colonne RP-18.

¹H-NMR (CDCl3): d 8,13 (d, 2H, OC(O) o-ArH), 7,75 (d, 2H, NC(O) o-ArH), 7,62 (t, 1H, OC(O) p-ArH), 7,53±7,49 (bande, 3H), 7,43±7,35 (bande, 7H), 6,91 (t, 1H, NH), 6,35 (s, 1H, C(10)-H), 6,27 (t, 1H, C(13)-H), 5,99 (t, 1H, C(3')-H), 5,69 (d, 1H, C(2)-H), 5,30 (dd,1H, C(7)-H), 5,44 (dd, 1H, C(2'-H), 5,20 (s, 5H, C(sq-H), 4,97 (d, 1H, C(5)-H) 4,39±4,25 (bande, 3H), 4,24±4,10 (bande, 7H), 3,96 (d,1H), 3,85 (dd, 1H), 3,75 (m, 1H), 3,42 (t, 2H, sq CH2-NH), 3,54 (m, 6H, CH2-O), 3,37 (m, 4H, O-CH2), 3,13 (m, 4H, CH2-N), 2,61 (p, 1H, C(6)-H et m 4H succinyle CH2), 2,46 (d, 3H, C(4)-OAc), 2,41 (m, 1H, C(14)-H), 2,29 et 2,20 (s 4H, CH2 sq. acide), 2,23(m, 1H, C(14)-H), 2,14 (d, 3H, C(10)-OAc), 2,01 (s, 3H, C(12)-CH3), 2,00 (m, 16H, CH2 sq), 1,97 (m, 1H, C(6)-H), 1,81 (s, 3H, C(8)-CH3), 1,71 (m, 18H, C(sq)-CH3), 1,41 (d, 3H), 1,21 (s,3H, C(15)-CH3), 1,16 (s, 3H, C(15)-CH3).

### Exemple 13

### Synthèse du squalènoylC22-paclitaxel

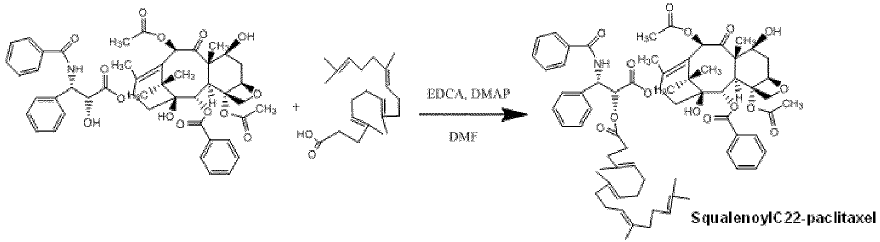

A une solution d'acide squalénique-C22 dans du DMF sec sont ajoutés de l'EDCA (1.5 eq.), du DMAP (0.5 eq.) et du paclitaxel (1.6 eq.) dans du DMF. Après 5 heures, la réaction est interrompue avec un pH d'eau égal à 5,0 et extrait à l'aide de saumure.

Le mélange brut est purifié par chromatographie sur SiO₂, élué avec du DCM/ acétate d'éthyle en utilisant un gradient optimisé. La fraction contenant le produit attendu est éluée avec 10 % d'acétate d'éthyle. Les fractions pures de squalénoylC22-paclitaxel sont collectées et la pureté est contrôlée par analyse HPLC sur une colonne RP-18, à polarité de phase inversée éluée avec un mélange acétonitrile/eau.

¹H-NMR (CDCl3): d 8,13 (d, 2H, OC(O) o-ArH), 7,75 (d, 2H, NC(O) o-ArH), 7,62 (t, 1H, OC(O) p-ArH),7,53±7,49 (bande, 3H), 7,43±7,35 (bande, 7H), 6,91 (t, 1H, NH), 6,35 (s, 1H, C(10)-H), 6,27 (t, 1H, C(13)-H), 5,99 (t, 1H, C(3')-H), 5,69 (d, 1H, C(2)-H), 5,30 (dd,1H, C(7)-H), 5,44 (dd, 1H, C(2'-H), 5,20 (s, 5H, C(sq-H), 4,97 (d, 1H, C(5)-H) 4,39±4,25 (bande, 3H), 4,24±4,10 (bande, 7H), 3,96 (d,1H), 3,85 (dd, 1H), 3,75 (m, 1H), 2,61 (p, 1H, C(6)-H), 2,46 (d, 3H, C(4)-OAc), 2,41 (m, 1H, C(14)-H), 2,29 et 2,35 (s 4H, CH2 sq. acide), 2,23(m, 1H, C(14)-H), 2,14 (d, 3H, C(10)-OAc), 2,01 (s, 3H, C(12)-CH3), 2,00 (m, 16H, CH2 sq), 1,97 (m, 1H, C(6)-H), 1,81 (s, 3H, C(8)-CH3), 1,71 (m, 15H, C(sq)-CH3), 1,41 (d, 3H), 1,21 (s,3H, C(15)-CH3), 1,16 (s, 3H, C(15)-CH3).

### Exemple 14

### Synthèse du 14-squalènoyl-doxorubicine

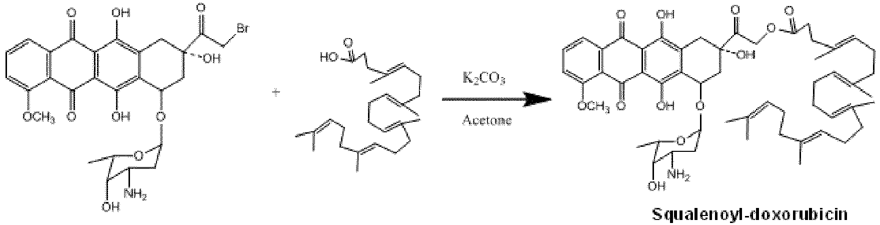

La doxorubicine en présence de brome et de triméthylorthoformiate, forme du 14-bromo-13-diméthyle acétate de doxorubicine, qui, sous agitation avec de l'acétone, fournit de la 14-bromodoxorubicine. La bromodoxorubicine dissoute dans de l'acétone sec est mise à réagir avec de l'acide squalénique (3 eq.) en présence de carbonate de potassium. Après 20 heures, la réaction est filtrée, le solvant est évaporé et le produit brut est purifié par chromatographie sur colonne SiO₂ (4:1, DCM-méthanol) afin d'obtenir de la 14-squalenoyl-doxorubicine.

¹H NMR (CDCl3): 8,02 (d, 1H, H-3), 7,87 (d, 1H, H-1), 7,70 (t, 1H, , H-2), 5,46 (s, 1H, H-10), 5,3- 5,25 (m, 2H, H-14a, H-14b) et 5,20 (s, 5H, C(sq-H) 5,19 (s, 1H, H-7), 4,15 (q, 1H, H-50), 4,01 (s, 3H, OCH3), 3,74 (m, 2H, H-30, H-40), 3,24 (d, 1H, H-10), 3,00 (d, 1H, H-10), 2,43 (m, 1H, H-8), 2,29 et 2,35 (s 4H, CH2 sq. acide), 2,13 (m, 1H, H-8), 2,03 (m, 16H, CH2 sq), 1,97 (m, 1H, H-20), 1,82 (m, 1H, H-20), 1,71 (m, 18H, C(sq)-CH3), 1,29 (d, 3H, CH3).

### Exemple 15

### Préparation et caractérisation de nanoparticules des dérivés synthétisés selon les exemples 10 à 14

Des nanoparticules de différents actifs sont préparées par nanoprécipitation. Dans le cas du paclitaxel, la solution éthanolique contenant le conjugué squanénoyl/paclitaxel (solution de base 5-10 mg/mL) est ajoutée, goutte-à-goutte, sous agitation (500 tpm) dans une solution de dextrose aqueuse à 5 %. La précipitation des nanoparticules survient spontanément. Les solvants organiques sont totalement évaporés à l'aide d'un Rotavapor® afin d'obtenir une suspension aqueuse de nanoparticules purs.

Concernant le squalénoyl-polyéthylène glycol (dans un ratio de 1:0.5), il est mélangé dans une solution éthanolique et les nanoparticules sont préparées selon la méthode précédemment décrite.

Pour la préparation de nanoparticules de squalénoyl-doxorubicine, on utilise un mélange de dichlorométhane et d'éthanol à la place de l'éthanol pur, mais le reste de la procédure est similaire à celle décrite ci-dessus.

La composition des nanoparticules formées à partir des conjugués précédemment décrits dans les exemples 10 à 14 est présentée dans le tableau ci-après :

| **Exemple No.** | **Ingrédients** | **Quantité** |
|---|---|---|
| 10 | Squalénoylsuccinyl-paclitaxel | 5,0 mg |
| | Squalénoyl-polyéthylène glycol (MM = 2369) | 2,5 mg |
| | Dextrose | 0,05 g |
| | Eau pour injection | q.s.p. 1 mL |
| 11 | Squalénoyldiglycolyl-paclitaxel | 5,0 mg |
| | Dextrose | 0,05 g |
| | Eau pour injection | q.s.p. 1 mL |
| 12 | Squalénoylsuccinyl-PEG340-paclitaxel | 3 mg |
| | Dextrose | 0,05 g |
| | Eau pour injection | q.s.p. 1 mL |
| 13 | SqualénoylC22-paclitaxel | 2,5 mg |
| | Dextrose | 0,05 g |
| | Eau pour injection | q.s.p. 1 mL |
| 14 | Squalénoyl-doxorubicine | 2 mg |
| | Dextrose | 0,05 g |
| | Eau pour injection | q.s.p. 1 mL |

La taille moyenne des nanoparticules et l'indice de polydispersité des nanoparticules sont déterminés à une température de 20 °C par diffusion de la lumière avec un zetasizer (Malvern Instruments, UK). Les mesures sont réalisées après dilution de la suspension des nanoparticules dans de l'eau MilliQ®.

La taille des nanoparticules des dérivés squalénoyle du paclitaxel et de la doxorubicine et leur indice de polydispersité sont listés dans le tableau ci-dessous :

| Exemple No. | Type de conjugués | Diamètre moyen des particules (nm) | Indice de polydispersité |
|---|---|---|---|
| 10 | Squalénoylsuccinyl-paclitaxel | 250,9 | 0,061 |
| 11 | Squalénoyldiglycolyl-paclitaxel | 150,0 | 0,075 |
| 12 | Squalenoylsuccinyl-PEG340-paclitaxel | 643,9 | 0,081 |
| 13 | SqualénoylC22-paclitaxel | 239,3 | 0,077 |
| 14 | Squalénoyl-doxorubicine | 138,2 | 0,325 |

### Exemple 16

### Evaluation de l'activité anti-cancer in vitro des conjugués squalénoyle

L'activité anti-cancer *in vitro* des conjugués du type squalénoyle/paclitaxel sous la forme de nanoparticules est réalisée sur une lignée cellulaire tumorale pulmonaire murine Madison's 109 (M109). Les cellules M109 sont cultivées dans RPMI 1640, supplémenté avec 10 % de sérum de veau fœtal, 50 U.ml⁻¹ de pénicilline et 50 µg.ml⁻¹ de streptomycine et 2 mM de L-glutamine. Cette analyse est réalisée en utilisant le test au bromure de 3-[4,5-diméthylthiazol-2-yl]-3,5-diphényle tétrazolium (MTT), mesurant l'activité déshydrogénase mitochondriale. Les cellules en phase de croissance exponentielle sont ensemencées sur plaque 96-puits et pré-incubées pendant 24 heures à 37 °C dans une atmosphère humidifiée avec 5 % de CO₂ dans l'air. Différentes dilutions de nanoparticules de paclitaxel sont ajoutées aux cellules dans le milieu de culture. Chaque dilution est testée trois fois. Après 72 heures à 37 °C, 200 µL de solution MTT dans le milieu de culture cellulaire (0,5 mg/ml) sont ajoutés dans chaque puits. Après incubation pendant 2 heures 30 à 37 °C, le milieu de culture est enlevé et les cristaux de formazan obtenus sont dissous dans 200 µL d'une solution d'extraction (diméthyle sulfoxide). Le pouvoir absorbant de la matière colorante transformée, qui est proportionnel au nombre de cellules viables, est mesuré à 570 nm en utilisant un lecteur de microplaques (Metertech Σ 960, Fisher Bioblock, Illkirch, France). Le pourcentage de cellules survivantes est calculé en faisant le ratio du pouvoir absorbant entre les cellules traitées et celles non traitées.

L'activité anti-cancer *in vitro* des nanoparticules squalénoyl-doxorubicine est évaluée sur une lignée cellulaire leucémique murine L1210 WT. Les cellules sont cultivées dans RPMI 1640 supplémenté avec 10 % de sérum de veau fœtal, 50 U.ml⁻¹ de pénicilline et 50 µg.ml⁻¹ de streptomycine et 2 mM de L-glutamine. L'évaluation est réalisée selon le protocole décrit ci-dessus.

Les concentrations d'inhibition 50 % (CI₅₀) des nanoparticules des différents conjugués de type squalénoyle/paclitaxel et squalénoyl-doxorubicine sont listées dans le tableau ci-dessous :

| Exemple No. | Type de nanoparticule | (CI₅₀) (µM) |
|---|---|---|
| 10 | Squalénoylsuccinyl-paclitaxel | 0,072 |
| 11 | Squalénoyldiglycolyl-paclitaxel | 0,175 |
| 12 | Squalénoylsuccinyl-PEG340-paclitaxel | 0,490 |
| 13 | SqualénoylC22-paclitaxel | 7,300 |
| 14 | Squalénoyl-doxorubicine | 0,291 |

### Exemple 17

### Evaluation de l'activité anti-cancer in vivo des nanoparticules d'un dérivé squalénoyldiglycolyl-paclitaxel obtenu selon l'Exemple 11

Des souris CD2F1 (âgées de 4-5 semaines) pesant environ 15-18 g ont été utilisées pour cette étude. Les souris sont nourries avec de la nourriture standard pour souris et de l'eau *ad libitum.* Le modèle tumorale sous-cutané M109 (tumeur pulmonaire murine) est développé en injectant les cellules M109 à croissance exponentielle (1x106 cellules) en suspension, sous la peau, dans la portion basse de l'abdomen des souris. Une tumeur palpable (environ 50-100 mm3) est laissée croître au site d'injection. Les souris porteuses de tumeur sont divisées en 2 groupes de 5-6, à savoir les non-traitées et les traitées avec des nanoparticules squalénoyldiglycolyl-paclitaxel 160 mg.kg⁻¹ (injectés en intraveineuse pendant 5 jours consécutifs). Les souris sont régulièrement contrôlées pour vérifier la différence du volume des tumeurs, et ainsi évaluer l'efficacité anti-cancer.

Les nanoparticules squalénoyldiglycolyl-paclitaxel démontrent une activité anti-cancer en contrôlant la progression des tumeurs M109 implantées hypodermiquement chez les souris.

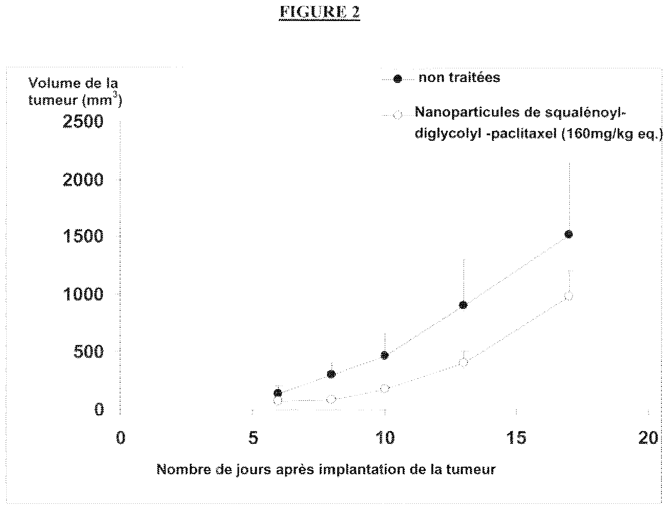

Ces résultats sont présentés en figure 2.

### Exemple 18

### Evaluation de l'activité anti-cancer in vivo des nanoparticules d'un dérivé squalénoyl-doxorubicine, obtenu selon l'Exemple 14

Des souris DBA/2 mice (âgées de 4 à 5 semaines) pesant environ 15-18 g ont été utilisées pour cette étude. Comme pour l'Exemple 17, les souris sont nourries avec de la nourriture standard pour souris et de l'eau *ad libitum.* Le modèle de leucémie métastasique agressive L1210 (une leucémie murine) est développé en injectant par voie intraveineuse les cellules à croissance exponentielle L1210 (0,1x10⁶ cellules) en suspension, dans les souris. Les souris porteuses de la tumeur sont divisées en 2 groupes de 5-6, à savoir les non traitées et les traitées avec des nanoparticules squalénoyl-doxorubicine (13 mg.kg⁻¹, injectées par voie intraveineuse aux jours 1, 7 et 14, après injection des cellules tumorales). Après traitement, les souris sont contrôlées régulièrement pour vérifier les différences de poids et le taux de survie, paramètres permettant d'évaluer l'efficacité de l'activité anti-cancer.

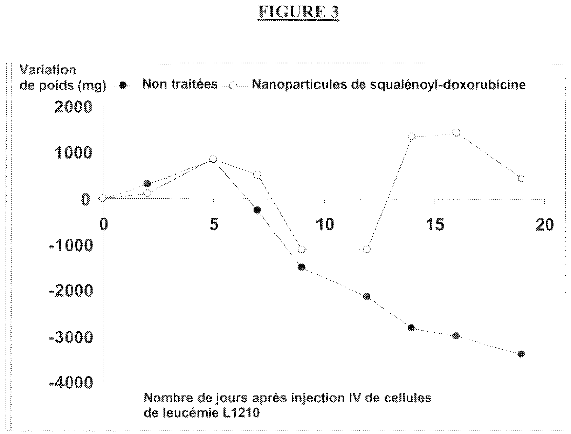

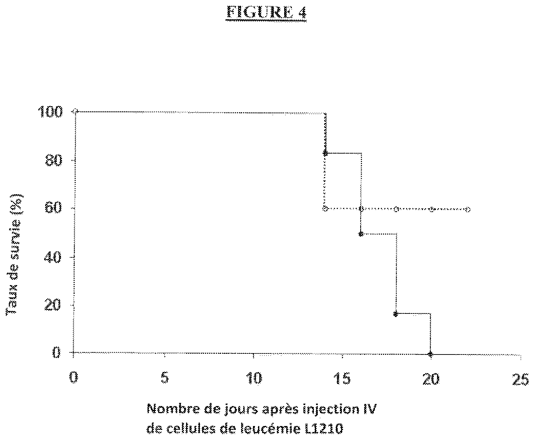

Dans ce modèle, qui représente la forme métastatique agressive de cancer, les nanoparticules de squalénoyl-doxorubicine démontrent une activité anti-cancer efficace (Figure 3) et un taux de survie des souris porteuses de leucémie amélioré (Figure 4).

## Revendications

1. Dérivé hydrodispersible d'un actif thérapeutique de faible solubilité aqueuse formé d'au moins une molécule dudit actif couplée de manière covalente à au moins une molécule d'un composé hydrocarboné à structure squalénique, ledit actif possédant une solubilité inférieure à 100 µg/ml dans de l'eau pure mesurée à température ambiante
et ledit actif étant un agent antitumoral chimiothérapeutique choisi parmi les taxoïdes, la doxorubicine et l'épirubicine.

2. Dérivé selon la revendication 1 dont ledit actif est choisi parmi le docétaxel et le paclitaxel.

3. Dérivé selon la revendication 1 ou 2 comprenant au moins deux radicaux à structure squalénique, identiques ou différents.

4. Dérivé selon l'une quelconque des revendications 1 à 3, dont ledit actif possède une solubilité inférieure à 25 µg/ml dans de l'eau pure mesurée à température ambiante, en particulier inférieure à 20 µg/ml voire inférieure à 10 µg/ml et plus particulièrement inférieure à 5 µg/ml.

5. Dérivé selon l'une quelconque des revendications précédentes, dans lequel le lien covalent existant entre ledit actif thérapeutique de faible solubilité aqueuse et une molécule d'un composé hydrocarboné à structure squalénique est figuré par un bras de liaison.

6. Dérivé selon la revendication précédente dans lequel le bras de liaison est choisi parmi les motifs (poly)aminoacides, polyols, saccharidiques, et polyéthylèneglycol (polyétheroxides) de faible poids moléculaire.

7. Dérivé selon l'une quelconque des revendications précédentes choisi parmi : 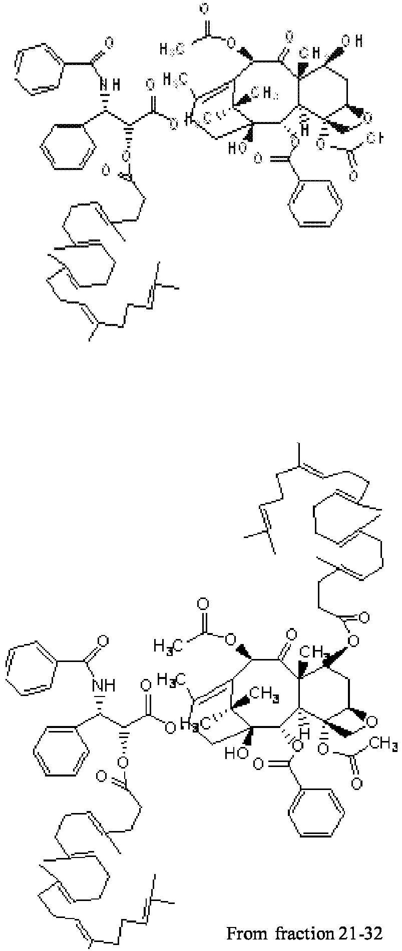 et

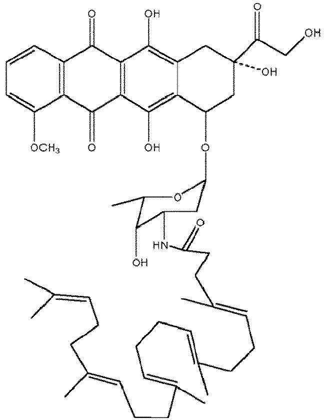

8. Nanoparticules hydrodispersibles d'au moins un actif thérapeutique de faible solubilité aqueuse dans lesquelles ledit actif y est présent sous une forme associée à au moins un composé hydrocarboné à structure squalénique formées à partir d'un dérivé selon l'une quelconque des revendications 1 à 7.

9. Nanoparticules selon la revendication 8, possédant une taille moyenne variant de 30 à 650 nm, en particulier de 30 nm à 500 nm, et en particulier de 50 à 250 nm, voire de 100 à 200 nm.

10. Dispersion aqueuse de nanoparticules selon l'une quelconque des revendications 8 à 9.

11. Procédé de préparation de nanoparticules selon l'une quelconque des revendications 8 à 9, **caractérisé en ce qu'**il comprend au moins :
- la solubilisation d'au moins un dérivé selon l'une quelconque des revendications 1 à 7 dans au moins un solvant organique à une concentration suffisante pour obtenir, lors de l'ajout du mélange correspondant, sous agitation, à une phase aqueuse, la formation instantanée de nanoparticules en suspension dans ladite phase aqueuse, et
- le cas échéant, l'isolement des dites nanoparticules.

12. Composition pharmaceutique comprenant à titre de matière active, au moins un dérivé selon l'une quelconque des revendications 1 à 7 ou des nanoparticules selon l'une quelconque des revendications 8 à 9 en association avec au moins un véhicule pharmaceutiquement acceptable.

## Patentansprüche

1. Wasserlösliches Derivat eines therapeutischen Wirkstoffes von geringer Wasserlöslichkeit, gebildet von mindestens einem Molekül des Wirkstoffes, das kovalent an mindestens ein Molekül einer Kohlenwasserstoffverbindung mit Skalenstruktur gekoppelt ist, wobei der Wirkstoff eine Löslichkeit unter 100 µg/ml in reinem Wasser, gemessen bei Raumtemperatur, besitzt, und wobei der Wirkstoff ein chemotherapeutisches Antitumormittel ist, ausgewählt unter den Taxoiden, Doxorubicin und Epirubicin.

2. Derivat nach Anspruch 1, dessen Wirkstoff unter Docetaxel und Paclitaxel ausgewählt ist.

3. Derivat nach Anspruch 1 oder 2, umfassend mindestens zwei Radikale mit Skalenstruktur, die identisch oder unterschiedlich sind.

4. Derivat nach einem der Ansprüche 1 bis 3, dessen Wirkstoff eine Löslichkeit unter 25 µg/ml in reinem Wasser, gemessen bei Raumtemperatur, insbesondere unter 20 µg/ml bzw. unter 10 µg/ml und genauer betrachtet unter 5 µg/ml besitzt.

5. Derivat nach einem der vorhergehenden Ansprüche, bei dem die kovalente Verbindung, die zwischen dem therapeutischen Wirkstoff mit geringer Wasserlöslichkeit und einem Molekül einer Kohlenwasserstoffverbindung mit Skalenstruktur besteht, durch einen Verbindungsarm verwirklicht ist.

6. Derivat nach dem vorhergehenden Anspruch, bei dem der Verbindungsarm unter den (Poly)aminosäure-, Polyol-, Saccharid- und Polyethylenglykol- (Polyetheroxid-) Motiven mit geringem Molekulargewicht ausgewählt ist.

7. Derivat nach einem der vorhergehenden Ansprüche, ausgewählt unter: 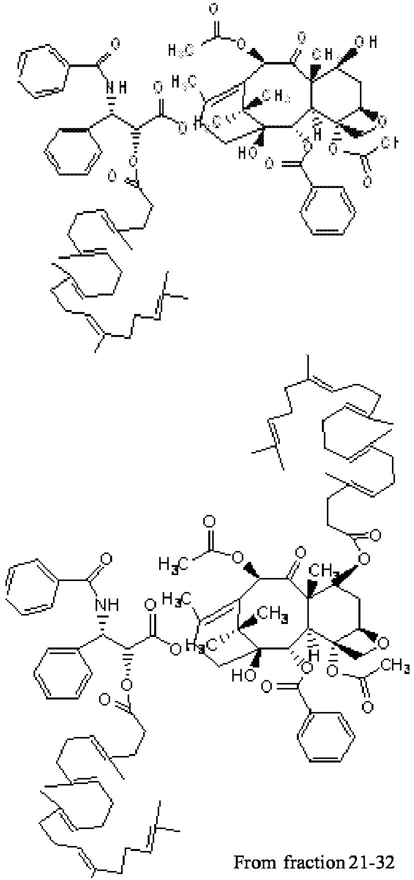 und

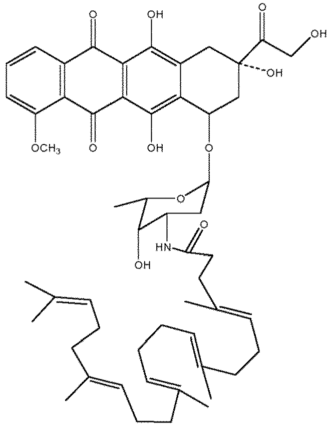

8. Wasserdispergierbare Nanopartikel mindestens eines therapeutischen Wirkstoffes mit geringer Wasserlöslichkeit, bei denen der Wirkstoff hier in einer Form vorhanden ist, die mindestens einer Kohlenwasserstoffverbindung mit Skalenstruktur zugeordnet ist, gebildet aus einem Derivat nach einem der Ansprüche 1 bis 7.

9. Nanopartikel nach Anspruch 8, die eine durchschnittliche Größe von 30 bis 650 nm, insbesondere von 30 bis 500 nm und insbesondere von 50 bis 250 nm bzw. von 100 bis 200 nm besitzen.

10. Wässrige Dispersion von Nanopartikeln nach einem der Ansprüche 8 bis 9.

11. Verfahren zur Herstellung von Nanopartikeln nach einem der Ansprüche 8 bis 9, **dadurch gekennzeichnet, dass** es mindestens umfasst:
- die Solubilisierung mindestens eines Derivats nach einem der Ansprüche 1 bis 7 in mindestens einen organischen Lösungsmittel mit einer ausreichenden Konzentration, um bei der Hinzufügung der entsprechenden Mischung bei Schütteln in einer wässrigen Phase die unmittelbare Bildung von Nanopartikeln in Suspension in der wässrigen Phase zu erzielen, und
- gegebenenfalls die Isolierung der Nanopartikel.

12. Pharmazeutische Zusammensetzung, umfassend als Wirkstoff mindestens ein Derivat nach einem der Ansprüche 1 bis 7 oder Nanopartikel nach einem der Ansprüche 8 bis 9 in Verbindung mit mindestens einem pharmazeutisch annehmbaren Träger.

## Claims

1. A water-dispersible derivative of a therapeutic active agent of low water solubility formed from at least one molecule of said active agent covalently coupled to at least one molecule of a hydrocarbon-based compound of squalene structure, said active agent having a solubility of less than 100 µg/ml in pure water, measured at room temperature,
and said active agent being a chemotherapeutic antitumor agent chosen from taxoids, doxorubicin and epirubicin.

2. The derivative as claimed in claim 1, said active agent of which is chosen from doxetaxel and paclitaxel

3. The derivative as claimed in claim 1 or 2, comprising at least two identical or different radicals of squalene structure.

4. The derivative as claimed in any one of claims 1 to 3, said active agent of which has a solubility of less than 25 µg/ml in pure water measured at room temperature, in particular less than 20 µg/ml or even less than 10 µg/ml and more particularly less than 5 µg/ml.

5. The derivative as claimed in any one of the preceding claims, in which the covalent linkage existing between said therapeutic active agent of low water solubility and a molecule of a hydrocarbon-based compound of squalene structure is embodied by a linker.

6. The derivative as claimed in the preceding claim, in which the linker is chosen from (poly)amino acid units, polyols, saccharides and polyethylene glycols (polyetheroxides) of low molecular weight.

7. The derivative as claimed in any one of the preceding claims, chosen from:

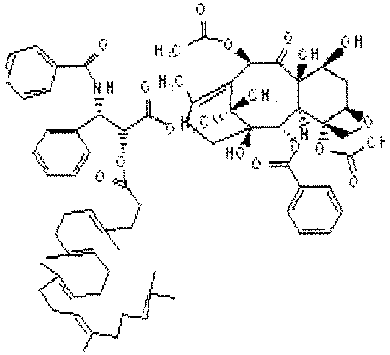

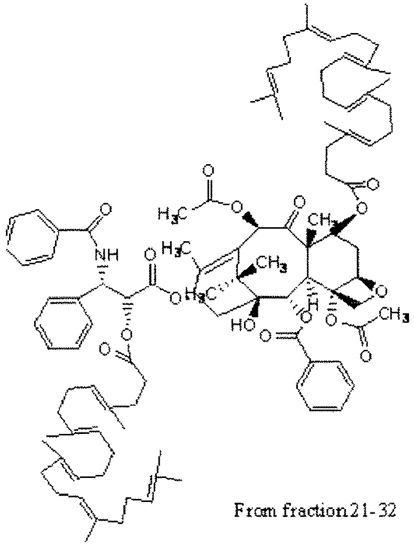

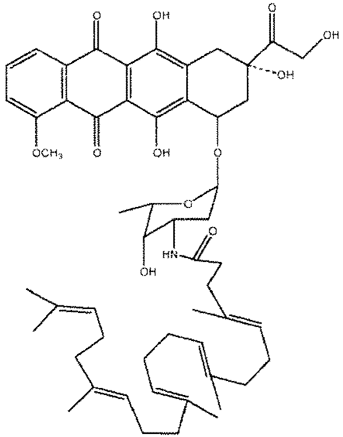

8. A water-dispersible nanoparticle of at least one therapeutic active agent of low water solubility in which said active agent is present therein in a form combined with at least one hydrocarbon-based compound of squalene structure formed from a derivative as claimed in any one of claims 1 to 7.

9. The nanoparticle as claimed in claim 8, having a mean size ranging from 30 to 650 nm, in particular from 30 to 500 nm and in particular from 50 to 250 nm, or even from 100 to 200 nm.

10. An aqueous dispersion of nanoparticles as claimed in any one of claims 8 to 9.

11. A process for preparing nanoparticles as claimed in any one of claims 8 to 9, **characterized in that** it comprises at least:
- solubilisation of at least one derivative as claimed in any one of claims 1 to 7 in at least one organic solvent at a concentration that is sufficient to obtain, during the addition of the corresponding mixture, with stirring, to an aqueous phase, the instantaneous formation of nanoparticles suspended in said aqueous phase, and
- where appropriate, the isolation of said nanoparticles.

12. A pharmaceutical composition comprising, as active material, at least one derivative as claimed in any one of claims 1 to 7 or nanoparticles as claimed in any one of claims 8 to 9 in combination with at least one pharmaceutically acceptable vehicle.
